# EUROPEAN PATENT SPECIFICATION

(11) **EP 0 713 399 B1**
(45) Date of publication and mention of the grant of the patent: **04.07.2007**
(21) Application number: 94908124.4
(22) Date of filing: 24.08.1992
(51) Int. Cl.: A61K 47/48, C08B 37/00, G01N 33/547

(54) **METHOD FOR BINDING TO POLYMERS**
VERFAHREN ZUM BINDEN AN POLYMERE
PROCEDE DE LIAISON A DES POLYMERES

(43) Date of publication of application: 29.05.1996
(73) Proprietor: INOVATA AB, S-161 71 Bromma (SE)
(72) Inventor: LINDGREN, Göran Einar Samuel, S-740 10 Almunge (SE)
(74) Representative: Lindgren, Anders
(86) International application number: PCT/SE1992/000573
(87) International publication number: WO 1994/004192

(56) References cited:
- EP-A- 0 087 786
- EP-A- 0 203 049
- US-A- 4 094 832

## Description

The present invention relates to a method of covalently binding a compound containing at least one nucleophilic group selected from amine or thiol to a polymer (carrier) which has a great number of amino and/or hydroxyl groups.

### TECHNICAL BACKGROUND

The products obtained with the method can be used in different methods of separation, i.e. liquid chromatography, preferably separation of biomolecules by ion-exchange chromatography, hydrophobic interaction chromatography and affinity chromatography. The products obtained with the method may also be of importance in size-exclusion chromatography.

During the past 20-30 years different bifunctional coupling reagents have been used to produce specific separation media for proteins, nucleic acids, polysaccarides and other biomolecules. By use of reagents like bisepoxides, epihalohydrins etc. bioaffine matrices showing antigen (hapten), antibody (and fragments showing antibody activity), Protein A, Protein G, IgG and lectin have been prepared. In analogy ion-exchange and metal ion adsorbents from inorganic and organic reagents has been prepared.

To bind biomolecules to soluble polymers and to use such products for separation by distribution between two liquid phases is also known.

Bifunctional reagents has also been used to prepare media for size-exclusion chromatography, by reaction of an organic or inorganic polymer containing hydroxyl groups and/or amino groups with a bifunctional cross-linking reagents like epihalohydrin, bishalohydrin and bisepoxides. By varying the degree of cross-linking matrices with different exclusion limits may be obtained.

A severe disadvantage with these reagents is that the reaction is difficult to control. Matrices with bridges and branches of unknown length and amount of hydroxyl groups may be obtained. The reagents may also be inactivated at the pH used during the reaction.

Matrices containing oxirane groups are moisture sensitive (hydrolysis). If they also contain hydroxyl or amino groups spontaneous cross-linking may occur during storage.

In a recent patent application US 4.973.683) a polymer containing hydroxyl groups is first reacted with an allyl halid or allyl glycidyl ether. The reaction is easily controlled towards expected amount of hydroxyl groups transferred to allyl ethers (example of an alkene group). In a second reaction HOX (X is equal to chlorine, bromine or iodine) is added to the allyl group giving halohydrin/oxirane groups which at suitable pH will react with adjacent hydroxyl groups in the polymer. The result is that the polymer is cross-linked in a defined way and the amount of side-reactions are minimized.

In an International search report compiled by the Swedish Patent Office EP 203049 (US 4,973,683), see above, EP 87786 and US 4,094,832 have been quoted as publications which define the level of the technique and classified to be without significant importance.

It has now been shown that alkene groups bound through a stable bridge (-B-) to a nitrogen atom or an oxygen atom in a polymer showing a great number of amino and/or hydroxyl groups in combination with activation of the alkene groups with HOX or X₂ can be used for coupling of different organic compounds. By choosing the right conditions cross-linking can be minimized. The amount of branches, that is hydroxyalkyl groups of varying length and cross-linking bridges will be low (nought).

### THE INVENTION

The invention is related to a method for coupling according to the introductory part at page 1.

A first characterization (A) of the invention is:
(i) that the polymer used in the invention shows a great number of alcoholic hydroxyl groups and/or amino groups where oxygen and/or nitrogen bind to a saturated carbon atom, and
(ii) that a great number of these hydroxyl groups and amino groups respectively are substituted with an inert and stable bridge -B- which binds directly to an alkene group. The atom of the bridge which bind to a nitrogen of the amine or an oxygen of the polymer is a saturated carbon atom.

A second characterization (B) is:
that in an aqueous media HOX or X₂, X is chlorine, bromine or iodine, is added at pH conditions such as the corresponding halohydrin, oxirane or vicinal dihalid is formed (activation).

A third characterization (C) is:
that after the addition reaction, the thus formed groups are allowed to react with a compound showing at least one nucleophilic group (Nu), which may be chosen among thiol (-SH) or amine. Thiol comprises thiolate (-S⁻) and that -SH binds to saturated or aromatic carbon atom. The amino group is alifatic or aromatic and may be a primary, secondary or tertiary amine. Side reactions are easiest to avoid for amine (primary amine) and thiol.

Solubility in water for the compound which reacts with the activated polymer is preferred.

The compound which is going to be used in step (C) always contains certain properties ascribed for the polymer. Hydrophobic compounds may be used to produce a hydrophobic polymer. For example benzyl amine, aminopentan, hexyl mercaptan etc. Hydrophilic compounds can be used to hydrophilize the polymer. An example is polyethylene glycol substituted in one end with an amino group and the other end with an ether. If the compound contains charged groups or groups that easily can be transformed to such groups it will give the polymer ion-exchange properties.

If the compound contains two functional groups (at least one nucleophilic group, see above) separated by an inert bridge (spacer) one of the groups can be utilized in step (C) and the other one for derivatization.

If the compound contains a chelating structure the invention gives a polymer which has affinity for metal ions. Chelating structures always contain at least two hetero atoms with free electron pairs. The most common hetero atoms are N, O and S. For O and S they are preferable negatively charged. The distance between two adjacent hetero atoms should be such that 5- or 6 membered rings will be formed when they in pairs coordinate a mutual metal ion.

This implies that in between two chelating hetero atoms should be two or three atoms, which often are carbon atoms (for example three saturated or two saturated and one sp² hybridized or one saturated and two sp² hybridized atoms). Also other atoms could be anticipated like phosphorous, oxygen and nitrogen. Normally, a chelating compound shows one or several groups - N((CH₂)ₙCh)₂ if necessary in combination with -N-(CH₂)ₙ-Ch', where n and n' is 1 or 2, and Ch and Ch' are chosen among -COO-. -PO₃²⁻ and -OPO₃²⁻. Further examples are crown ethers.

Likewise the compound can be one of the parts in a bioaffine pair such as anti-gen/hapten and antibody/antibody active fragment; Protein A,G or L and IgG or an IgG specific fragment; carbohydrate and lectin; biotin and avidin/streptavidin; enzyme and substrate/co-enzyme/inhibitor; hormone and receptor etc. This type of compounds are often biopolymers with polypeptide-/oligopeptide-, polysaccharide-/oligosaccharide- or polynucleotide-/oligonucleotide structure. The compounds may be derivatized.

The polymer may be inorganic or organic (synthetic polymer or biopolymer). The hydroxyl group may directly bound to or be part of the monomer unit or indirectly via organic bridges (spacers). The same applies to amino groups which can be primary or secondary. The hydroxyl groups as well as the amino groups are preferable bound directly to saturated carbon atoms.

The most important inorganic polymer is based on silica (for example Kromasil, EKA-Nobel, Sweden). Activated silica reagents are often used for coupling to silica gels.

Examples on synthetic organic polymers are polyacrylat, polymetacrylat, polyacrylamide, polymetacrylamide etc. which is O- or N-substituted with hydroxyalkylgroups (C₂-C₄) and if necessary also cross-linked via for example O,O'-/N, N'-alkene or O,O'-/N,N'-hydroxyalken. Other examples are polyvinyl alcohol which can be derivatized or cross-linked, hydrophilized polystyrene- or polystyrene/divinylbensen, hydrophilized trimethylolpropantrimethacrylate etc. Biopolymers have a backbone of biological origin with analogous repeating units. Generally they have a polysaccharide as basic structure. Important such biopolymers are cellulose, starch, dextran, agar (agarose) etc., which may be cross-linked or derivatized in the former described way. Commercial available biopolymers are Sephadex and Polydex (cross-linked dextran in a beaded form), Pharmacia Biotechnology Group, Sweden and Polydex Biological, Canada, respectively. Novarose and Sepharose Cl (cross-linked agarose in a beaded form), Inovata AB, Sweden and Pharmacia Biotechnology Group, Sweden, respectively.

The polymer may be soluble or insoluble in water. In many cases the polymer is originally soluble in water but after derivatization insoluble in water (i.e. Senhadex).

Insoluble polymers may be of different geometrical and physical form. Examples are paper, beads, layers (if necessary on a support matrix), foam etc. To increase the available surface area towards the surrounding media the insoluble polymer generally forms a porous matrix.

That the polymer contains alkene groups refers to the hydroxy or amine hydrogen in the basic polymer being substituted with groups containing one or several isolated carbon-carbon double bonds which carbon atoms only bind to hydrogen, saturated or aromatic carbon atom. The preferred groups containing alkene can be denoted

-B-CH = CH₂ (I)

-B- is a hydrocarbon chain which is straight, branched or cyclic with 1-15 carbon atoms, preferable at the most 10 and generally below 5.

The hydrocarbon chain may be substituted with one or several amino- and/or hydroxyl groups. In the chain one or several nitrogen and/or oxygen atoms may be included (part of secondary amine and ether respectively). In certain specific cases a carbon atom may be replaced by a silicon atom. In general all carbon atoms in -B- are saturated. From a synthetic point of view it is preferred that -Bis equal to methylene (-CH₂).

Polymers containing said alkene groups have been described earlier. US-A-4, 973,683 and GB 1,511,969 (soluble polymer; allyldextran). The most appropriate production method is by reaction of a basic polymer containing a hydroxyl group or amino group (i.e. P-OH or P-NH₂, where P is a part of the basic polymer) with a compound of the structure

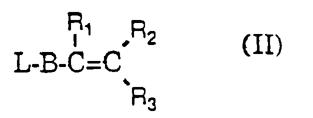

At the reaction L is substituted by P-O (alternatively P-NH). B is earlier said bridge. R₁, R₂, and R₃ is chosen between hydrogen, shorter alkyl, aryl which may be substituted by shorter alkyl. L is a leaving group, in general a weak nucleophile like iodine, bromine, chlorine, p-toluene sulphonate, groups like oxirane, halohydrin and vicinal dihalid etc.

Shorter alkyl refers foremost to C₁-C₆ alkyl and aryl refer foremost to phenyl.

The groups in formula II are chosen in a way that they are compatible to each other, that is during such conditions that L is not reacting with the other groups. In general, for practical, economical and synthetic reasons R₁ = R₂ = R₃ = H and/ or -B- are equal to -CH₂-.

The substitution reaction normally takes place at strong alkaline pH and during such conditions that side reactions practically do not occur.

The contents of alkene group (C=C) of the polymer may vary more widely and are usually determined by what is going to be coupled and the method of application or the final product. A suitable range is often 0.01-3 mmol/g dry gel (polymer).

The alkene groups of the polymer are reacted through addition of HOX or X₂ (characteristicum B), whereupon formed halohydrin or vicinal dihalid react with the compound containing the nucleophilic group. If possible the reaction occur through an intermediate formation of oxirane. pH is important and varies with choice of X and nucleophilic group of the compound. At too high pH HOX disproportionate and will be consumed (side reaction). At too low pH the halogen will exist as X₂. To summarize, pH as a rule should be 5-8 besides that the extremes in the interval should be avoided for iodine. Below pH 5 mainly X₂ is added and furthermore there is a risk for oxidation if the polymer is a polysaccharide. Preferred halogens are bromine and chlorine.

After the reaction of the alkene groups with HOX or X₂ the pH is adjusted to neutral with alkali. Addition of HOX is done to permanent colour which then will disappear during neutralization. The halogen functions as an indicator. If necessary the adduct is washed before the compound containing the nucleophile is added.

pH is important. If the compound contains a thiol group the reaction normally comes to an end in neutral environment with an alkaline shift (pH 6-8). As a rule amines can be used without a buffer. The pH value of the amine is normally acceptable (pH 7-11).

Extreme pH values according to characteristicum C means that formed halohydrin or vicinal dihalid easily can react through oxirane with water (hydrolysis) or with adjacent hydroxyl groups (cross-linking). Most sensitive is the reaction for X = iodine. In the pH interval 7-11 potassium iodine may in certain cases catalyse the reaction to increase the yield of the compound.

One aspect of the invention is the final product, that is the polymer containing the bound compound. This is characterized by being well defined (uniform) with reference to cross-linking and spacers (free and bound to the compound) of different length are missing.

Another aspect of the invention is the use of the allyl polymer for activation and coupling mentioned above.

The invention is illustrated by following examples. The invention is more closely defined in enclosed claims which are part of the description.

### Example 1.

1.5 g of dry cellulose (Munktells filter paper no. OOH S1-80-40) was allowed to swell in 50 g 1 M sodium hydroxide for three hours in presence of 1 ml 14.3 M sodium borohydride. 3.4 ml allyl bromide was added and the reaction mixture was stirred overnight. The activated cellulose was washed with water until neutral pH and carbonate buffer pH 6 was added. Saturated bromine water was added until the colour of the solution was slightly yellow. The mixture was filtered on a glass filter and washed with water.

To the activated cellulose 60 ml of water and 10 g of trimethyl amine was added. Reaction overnight. Elemental analysis of nitrogen resulted in 0.55 %.

### Example 2.

1.5 g of cellulose (Munktells filter paper no. OOH S1-80-40) was activated according to example 1. To filtered and washed cellulose 60 ml of water and 0.5 g 1 M sodium hydroxide was added followed by 2 g of mercapto ethanol. Reaction overnight. The amount of sulphur was 0.37 % (elemental analysis).

### Example 3.

1.5 g dried cross-linked dextran beads (PDX Biologicals, Toronto, Canada) were allowed to swell for two hours in 50 g 1 M NaOH in presence of 1 ml 14.3 M sodium borohydrid. Then 3.4 ml allyl bromide was added and the mixture was stirred overnight. The dextran beads were washed until neutral pH and suspended in carbonate buffer pH 6.5. Saturated bromine water was added until a slight yellow colour remained. The activated beads were washed on a glass filter and suspended in 30 ml of water. 5 g of trimethyl amine was added.

Reaction overnight whereafter the excess of trimethyl amine was washed away and the amount of nitrogen was determined by elemental analysis. Result 2.0 % of nitrogen.

### Example 4.

Performed according to example 3 but the allyl bromide was replaced by 4.5 g of allyl glycidyl ether. Addition of bromine water followed by mercapto ethanol gave 0.8 % of sulphur (elemental analysis).

### Example 5.

Performed according to example 3 but trimethyl amine was replaced by mercapto ethanol. Elemental analysis resulted in 1.45 % of sulphur.

### Example 6.

2 gram of Kromasil^{™}-100Å, 10 µm (EkaNobel, Surte, Sweden) was washed with acetone and dried by water suction. The silica acid was suspended in 25 ml dried toluene and 2 ml of allylchlorodimethyl silane and 1 ml of pyridine was added. The mixture was allowed to react at 50 °C overnight. After water suction and washing on a glass filter the allylated silica gel was suspended in 20 ml of water and saturated bromine water was added until the mixture was slightly yellow. The amount of bromine consumed corresponded to 250 µmol/g silica.

### Example 7.

100 ml of beaded agarose gel (Novarose^{™} SE-1200, 15-20 µm, Inovata AB, Sollentuna, Sweden) was suspended i 100 ml 5 M sodium hydroxide in presence of sodium borohydride and 12 g allyl bromide was added. Reaction overnight whereafter the gel was washed with water until neutral pH. To 10 ml of the activated gel an excess of trimethyl amine was added and allowed to react during stirring overnight. The amount of nitrogen was 1.98 % (determined by elemental analysis).

The remaining activated gel was stored in water at neutral pH and in room temperature. During a period of six month samples were taken out and reacted with excess of trimethyl amine and the amount of nitrogen was determined by elemental analysis. The result is shown in the table below. The result shows that the decrease in activity during a six month period is negligeable.

| Days | % N |
|---|---|
| 0 | 1,96 |
| 38 | 1,87 |
| 73 | 1,83 |
| 138 | 1,80 |
| 180 | 1,79 |

### Example 8.

Activated agarose prepared according to example 7 was suspended in carbonate buffer of pH 8 and pH 9, respectively. Bovine serum albumin was added and allowed to react overnight. Result 0.19 % and 0.37 % nitrogen which correspond to about 1 respectively 2 mg protein /ml gel.

## Claims

1. A method of binding an organic compound to the surface of a polymer or cross-linked polymer matrix, said organic compound comprising at least one nucleophilic group, the polymer being selected from the group consisting of cellulose, cross-linked dextran beads, silica gel, agarose gel, and synthetic polymers, said polymer matrix comprising amino and/or hydroxyl groups, and said matrix being selected from the group consisting of paper, beads, supported or non-supported layers, and foam, wherein the amine nitrogen and hydroxy oxygen respectively of the polymers in said polymer matrix, binds to a saturated carbon atom, said amino groups being primary or secondary amines, and wherein said polymer exhibits a plurality of alkene groups, each of which binding to the polymer through a stable and inert bridge (-B-), wherein -B- is a carbon chain having 1-15 carbon atoms and which is straight, branched or cyclic, and wherein the carbon chain may be substituted with at least one amino and/or hydroxyl group and/or interrupted by at least one amino nitrogen and/or oxygen atom, and a carbon atom in the chain may be replaced by a silicon atom, where said bridge B substitutes a hydrogen in an amine or hydroxy group in said polymers;
wherein said method comprises the following steps:
reacting the polymer matrix in an aqueous environment and at pH 4-8 with HOX, or X₂, wherein X is chlorine, bromine or iodine such that the double bonds of the alkene groups are converted into oxirane, vicinal dihalid or halohydrin moieties, and
reacting the formed oxirane, vicinal dihalid or halohydrin groups with said organic compound comprising at least one nucleophilic group,
**characterised in that** said nucleophilic group(s) is selected from one member of the group consisting of amines and thiols, and that said nucleophilic substitution is performed at a pH in the range of 7-11 when the nucleophile is an amine, and 6-8 when the nucleophile is a thiol, such that said organic compound binds only to the carbon atoms in the structure of the oxirane, vicinal dihalid or halohydrin moieties.

2. A method according to claim 1, **characterised in that** said alkene is -CH=CH₂.

3. A method according to any of claims 1-2, **characterised in that** at most one, of the at least one oxygen and/or nitrogen atom of the carbon chain of-B-, binds to the same carbon atom of -B-.

4. A method according to any of claims 1-3, **characterised in that** -B- contains at the most 10 nitrogen or oxygen atoms.

5. A method according to any of claims 1-3, **characterised in that** -B- contains less than 4 nitrogen or oxygen atoms.

6. A method according to any of claims 1-5, **characterised in that** -B- is methylene.

7. A method according to any of claims 1-6, **characterised in that** the organic compound is a biopolymer selected from proteins (polypeptides), nucleic acids, oligopeptides, oligonucleotides, and derivatives thereof where the polymeric structure is intact.

8. A method according to any of claims 1-7, **characterised in that** the organic compound is one of the components in a bioaffine pair.

9. A method according to claim 8, **characterised in that** said one of the components in a bioaffine pair, is an anti-body or anti-body active fragment or Fc-fragment thereof, an antigen or hapten, Protein A or Protein G or IgG binding fragments of those, lectin, a compound containing a carbohydrate structure, biotin or (strep)avidin.

10. A method according to any of the claims 1-9, **characterised in that** the polymer contains 0.1-3 mmol alkene groups (C=C) per ml dry polymer exhibiting alkene groups.

## Patentansprüche

1. Verfahren zur Bindung einer organischen Verbindung an die Oberfläche eines Polymers oder einer vernetzten Polymer-Matrix, wobei die organische Verbindung mindestens eine nukleophile Gruppe umfasst, das Polymer aus der Gruppe bestehend aus Cellulose, Perlen von vernetztem Dextran, Silicagel, Agarosegel und synthetischen Polymeren ausgewählt ist, die Polymer-Matrix Amino- und/oder Hydroxylgruppen umfasst und die Matrix aus der Gruppe bestehend aus Papier, Perlen, geträgerten oder nichtgeträgerten Schichten und Schaum ausgewählt ist, wobei der Amin-Stickstoff bzw. Hydroxy-Sauerstoff der Polymere in der Polymer-Matrix an ein gesättigtes Kohlenstoffatom bindet, die Aminogruppen primäre oder sekundäre Amine sind, und wobei das Polymer eine Mehrzahl von Alkengruppen aufweist, von denen jede über eine stabile und inerte Brücke (-B-) an das Polymer bindet, wobei -B- eine Kohlenstoffkette mit 1-15 Kohlenstoffatomen ist und geradkettig, verzweigt oder cyclisch ist, und wobei die Kohlenstoffkette durch mindestens eine Amino- und/oder Hydroxylgruppe substituiert sein kann und/oder unterbrochen durch mindestens ein Amino-Stickstoff- und/oder Sauerstoffatom, und ein Kohlenstoffatom in der Kette durch ein Siliciumatom ersetzt sein kann, wobei die Brücke B einen Wasserstoff in einer Amin- oder Hydroxygruppe in den Polymeren ersetzt;
wobei das Verfahren die folgenden Schritte umfasst:
Umsetzen der Polymer-Matrix in einer wässerigen Umgebung und bei einem pH-Wert von 4-8 mit HOX oder X₂, wobei X Chlor, Brom oder Iod ist, so dass die Doppelbindungen der Alkengruppen in Oxiran-, vicinale Dihalogenid- oder Halohydrin-Gruppierungen überführt werden, und
Umsetzen der gebildeten Oxiran-, vicinalen Dihalogenid- oder Halohydrin-Gruppen mit der organischen Verbindung, die mindestens eine nukleophile Gruppe umfasst,
**dadurch gekennzeichnet, dass** die nukleophile(n) Gruppe(n) ausgewählt ist/sind aus einem Mitglied der Gruppe, die aus Aminen und Thiolen besteht, und dass die nukleophile Substitution bei einem pH-Wert im Bereich von 7-11 durchgeführt wird, wenn das Nukleophil ein Amin ist, und im Bereich 6-8, wenn das Nukleophil ein Thiol ist, so dass die organische Verbindung nur an die Kohlenstoffatome in der Struktur der Oxiran-, vicinalen Dihalogenid- oder Halohydrin-Gruppierungen bindet.

2. Verfahren nach Anspruch 1, **dadurch gekennzeichnet, dass** das Alken -CH=CH₂ ist.

3. Verfahren nach irgendeinem der Ansprüche 1-2, **dadurch gekennzeichnet, dass** höchstens eines von dem mindestens einen Sauerstoff- und/oder Stickstoffatom der Kohlenstoffkette von -B- an dasselbe Kohlenstoffatom von -B- bindet.

4. Verfahren nach irgendeinem der Ansprüche 1-3, **dadurch gekennzeichnet, dass** -B- höchstens 10 Stickstoff- oder Sauerstoffatome enthält.

5. Verfahren nach irgendeinem der Ansprüche 1-3, **dadurch gekennzeichnet, dass** -B- weniger als 4 Stickstoff- oder Sauerstoffatome enthält.

6. Verfahren nach irgendeinem der Ansprüche 1-5, **dadurch gekennzeichnet, dass** -B- Methylen ist.

7. Verfahren nach irgendeinem der Ansprüche 1-6, **dadurch gekennzeichnet, dass** die organische Verbindung ein Biopolymer ist, ausgewählt aus Proteinen (Polypeptiden), Nukleinsäuren, Oligopeptiden, Oligonukleotiden und Derivaten davon, wobei die polymere Struktur intakt ist.

8. Verfahren nach irgendeinem der Ansprüche 1-7, **dadurch gekennzeichnet, dass** die organische Verbindung eine der Komponenten in einem bioaffinen Paar ist.

9. Verfahren nach Anspruch 8, **dadurch gekennzeichnet, dass** in eine der Komponenten in einem bioaffinen Paar ein Antikörper oder ein als Antikörper aktives Fragment oder Fc-Fragment eines Antikörpers, ein Antigen oder Hapten, Protein A oder Protein G oder IgG-bindende Fragmente davon, Lektin, eine Verbindung, die eine Kohlenhydratstruktur enthält, Biotin oder (Strept)avidin darstellt.

10. Verfahren nach irgendeinem der Ansprüche 1-9, **dadurch gekennzeichnet, dass** das Polymer 0,1-3 mmol Alkengruppen (C=C) pro ml trockenem Polymer, das Alkengruppen aufweist, enthält.

## Revendications

1. Procédé pour lier un composé organique à la surface d'un polymère ou d'une matrice polymère réticulée, ledit composé organique comprenant au moins un groupe nucléophile, le polymère étant choisi dans le groupe consistant en la cellulose, des perles de dextran réticulé, le gel de silice, le gel d'agarose et des polymères synthétiques, ladite matrice polymère comprenant des groupes amines et/ou hydroxyles, et ladite matrice étant choisie dans le groupe consistant en du papier, des perles, des couches supportées ou non supportées, et une mousse, dans lequel respectivement l'azote de l'amine et l'oxygène de l'hydroxyle des polymères dans ladite matrice polymère se lient à un atome de carbone saturé, lesdits groupes amines étant des amines primaires ou secondaires, et dans lequel ledit polymère présente une pluralité de groupes alcènes, chacun d'eux se liant au polymère par un pont inerte et stable (-B-) dans lequel -B- est une chaîne carbonée ayant 1 à 15 atomes de carbone et qui est linéaire, ramifiée ou cyclique, et dans lequel la chaîne carbonée peut être substituée avec au moins un groupe amine et/ou hydroxyle et/ou interrompue par au moins un azote d'amine et/ou un atome d'oxygène, et un atome de carbone dans la chaîne peut être remplacé par un atome de silicium, où ledit pont B se substitue à un hydrogène dans un groupe amine ou hydroxyle dans lesdits polymères,
dans lequel ledit procédé comprend les étapes suivantes :
réaction de la matrice polymère dans un environnement aqueux et à un pH de 4-8 avec HOX ou X₂, où X est du chlore, du brome ou de l'iode de telle sorte que les doubles liaisons du groupe alcène soient converties en fragments oxiranes, dihalogénures vicinaux ou halohydrines, et
réaction des groupes oxiranes, dihalogénures vicinaux ou halohydrines formés avec ledit composé organique comprenant au moins un groupe nucléophile,
**caractérisé en ce que** le ou lesdits groupes nucléophiles est/sont choisi(s) parmi un membre du groupe composé des amines et des thiols, et **en ce que** ladite substitution nucléophile est effectuée à un pH dans la plage de 7 à 11 quand le nucléophile est une amine, et de 6 à 8 quand le nucléophile est un thiol, de sorte que ledit composé organique se lie uniquement aux atomes de carbone dans la structure des fragments oxiranes, dihalogénures vicinaux ou halohydrines.

2. Procédé selon la revendication 1, **caractérisé en ce que** ledit alcène est -CH=CH₂.

3. Procédé selon l'une quelconque des revendications 1 à 2, **caractérisé en ce qu'**au maximum un du au moins un atome d'oxygène et/ou d'azote de la chaîne carbonée -B-se lie au même atome de carbone de -B-.

4. Procédé selon l'une quelconque des revendications 1 à 3, **caractérisé en ce que** -B- contient au maximum 10 atomes d'azote ou d'oxygène.

5. Procédé selon l'une quelconque des revendications 1 à 3, **caractérisé en ce que** -B- contient moins de 4 atomes d'azote ou d'oxygène.

6. Procédé selon l'une quelconque des revendications 1 à 5, **caractérisé en ce que** -B- est un méthylène.

7. Procédé selon l'une quelconque des revendications 1-6, **caractérisé en ce que** le composé organique est un biopolymère choisi parmi des protéines (polypeptides), des acides nucléiques, des oligopeptides, des oligonucléotides, et leurs dérivés où la structure polymérique est intacte.

8. Procédé selon l'une quelconque des revendications 1 à 7, **caractérisé en ce que** le composé organique est un des composants dans une paire bioaffine.

9. Procédé selon la revendication 8, **caractérisé en ce que** ledit un des composants d'une paire bioaffine est un anticorps ou un fragment actif d'anticorps ou un fragment Fc de celle-ci, un antigène ou un haptène, une protéine A ou protéine G ou des fragments se liant à une IgG de ces derniers, une lectine, un composé contenant une structure de sucre, la biotine ou la (strep)avidine.

10. Procédé selon l'une quelconque des revendications 1 à 9 **caractérisé en ce que** le polymère contient 0,3-1 mmol de groupes alcènes (C=C) par ml de polymère sec présentant des groupes alcènes.
